# FASCICULE DE BREVET EUROPEEN

(11) **EP 0 653 226 B1**
(45) Date de publication et mention de la délivrance du brevet: **05.09.2001**
(21) Numéro de dépôt: 94402591.5
(22) Date de dépôt: 16.11.1994
(51) Int. Cl.: A61N 1/39

(54) **Appareil médical, notamment défibrillateur implantable, à fonctions d'enregistrement Holter intégrées**
Medizinisches Gerät, insbesondere implantierbarer Defibrillator, mit integrierten Aufzeichnungsfunktionen von Holter
Medical device, in particular implantable defibrillator, with integrated Holter's recording functions

(30) Priorité: 17.11.1993 FR 9313734
(43) Date de publication de la demande: 17.05.1995
(73) Titulaire: ELA MEDICAL (Société anonyme), F-92541 Montrouge (FR)
(72) Inventeur: Jacobson, Peter, F-67500 Haguenau (FR); Kroiss, Daniel, F-67500 Schweiehouse/Moder (FR)
(74) Mandataire: Dupuis-Latour, Dominique

(56) Documents cités:
- EP-A- 0 339 471
- EP-A- 0 487 429
- US-A- 4 295 474
- US-A- 4 374 382
- US-A- 4 513 743
- US-A- 4 548 209
- US-A- 5 052 399

## Description

La présente invention concerne les fonctions de traitement et d'enregistrement des données relatives à l'activité cardiaque, également appelées "fonctions Holter".

L'invention concerne notamment les fonctions Holter mises en oeuvre par des appareils implantés qu'il est possible d'interroger ultérieurement par télémétrie (au sens ou ce mot est entendu dans le domaine de la stimulation cardiaque, c'est-à-dire la transmission sans fil d'informations entre un appareil implanté et un dispositif externe par ondes) au moyen d'un appareil externe appelé "programmateur", ces fonctions étant alors connues sont le nom de "fonctions Holter implantables".

Bien que dans la présente description on se référera principalement aux fonctions Holter gérées par des appareils implantés tels que stimulateur, cardioverteur et défibrillateur implantés, l'invention peut être également transposée au cas des appareils Holter simples ayant pour seule fonction la surveillance et l'enregistrement de l'activité cardiaque.

De la même façon, bien que l'on se référe principalement aux signaux cardiaques mesurés au moyen d'électrodes implantées ("électrogrammes"), les fonctions mises en oeuvre par l'invention peuvent être également appliquées à des signaux mesurés par des électrodes externes ("électrocardiogrammes").

Les fonctions Holter, notamment implantables, constituent un sujet étudié de longue date et ont fait l'objet de formes très variées de mise en oeuvre.

Le US-A-4 295 474 (Fischell) décrit un système d'enregistrement d'électrogramme mémorisant numériquement, dans une première mémoire, l'électrogramme couvrant une période précédant immédiatement et incluant la survenance d'un certain événement physiologique, et, dans une deuxième et une troisième mémoires, des compteurs d'épisodes de fibrillation et de chocs de défibrillation couvrant une seconde période suivant l'événement. Ce système mémorise de façon continue l'électrogramme et le "gèle" lorsque survient l'événement déclenchant, ou à un certain moment prédéterminé après l'événement déclenchant.

Au sixième Symposium mondial sur la stimulation cardiaque qui s'est tenu à Montréal du 2 au 5 octobre 1979, Attuel, Mugica et coll. avaient présenté un stimulateur implantable avec une fonction Holter qui comptait les pauses dans le rythme cardiaque. Ils proposaient un système à venir, qui identifierait les bradycardies ou les tachycardies et enregistrerait les signaux auriculaires et ventriculaires en cas de détection de ces arythmies.

Dans une publication intitulée "Technologie des mémoires et fonction Holter implantable" présentée à la conférence *Cardiostim* en octobre 1980, Ripart et Jacobson ont exposé le partitionnement de la mémoire Holter d'un stimulateur implantable. Le stimulateur comportait un détecteur pour classer les arythmies, et trois schémas de mémorisation des information étaient proposés :
1°) un électrogramme de deux secondes déclenché par un type choisi d'arythmie détectée ;
2°) des ensembles de compteurs d'occurrences pour des types choisis d'arythmies, chaque ensemble correspondant à une période temporelle de l'historique de l'appareil ;
3°) des histogrammes d'intervalles cardiaques, chaque histogramme correspondant également à une période temporelle.

Le US-A-4 374 382 (Markowitz) décrit un système de "télémétrie par marqueur d'événement". Les marqueurs consistent en codes numériques correspondant à des événements cardiaques tels que détection auriculaire, stimulation ventriculaire, etc. L'appareil émet les codes par télémétrie vers un récepteur externe lorsque les événements correspondants surviennent. Mais cet appareil ne mémorise pas durablement les codes des marqueurs dans l'appareil implanté pour affichage éventuel à un moment souhaité par l'utilisateur.

Le US-A-4 513 743 (Van Arragon et coll.) décrit un stimulateur implantable produisant des histogrammes d'intervalles cardiaques correspondant chacun à une période de temps donnée, d'une façon semblable au concept présenté pour la première fois par Ripart et Jacobson en 1980 (*supra*).

Le US-A-5 052 399 (Olive et coll.) décrit un procédé pour coder et comprimer un enregistrement de longueurs consécutives de cycles cardiaques, dans lequel l'appareil mémorise les différences de longueurs des cycles successifs ou, en cas de dépassement de capacité d'un mot de mémoire, mémorise la longueur du dernier cycle.

Le stimulateur "Chorus II" commercialisé par la Société ELA Medical depuis novembre 1991 comporte une fonction Holter implantable appelée "chaîne de marqueurs". L'appareil mémorise un marqueur puis une information permettant de déterminer l'instant de survenance du marqueur par rapport à l'événement marqué précédent. Il mémorise une chaîne de marqueurs ainsi datés, permettant à un appareil externe de reconstruire un chronogramme synthétisé des événements marqués, avec les intervalles séparant ceux-ci.

Comme on peut le voir, des progrès multiples ont ainsi été accomplis dans le sens d'une mémorisation automatique des informations liées à l'arythmie dans les appareils cardiaques implantables, principalement les stimulateurs simples (sans fonction de cardioversion ou de défibrillation).

Toutefois, en raison de la capacité mémoire limitée, tous les systèmes de ce type impliquent un dilemme entre le souhait de mémoriser des informations détaillées sur chacun des battements cardiaques et celui de mémoriser des informations couvrant des périodes de temps étendues, ou couvrant de nombreux épisodes d'arythmie. En général, plus le niveau de détail est élevé, plus court sera l'enregistrement susceptible de tenir dans un espace mémoire donné.

Ce dilemme est le même, par analogie, que celui auquel sont confrontés les utilisateurs d'appareils photo possédant plusieurs objectifs, dont la dimension du champ de vision diminue lorsque la focale plus longue procure plus de détails.

Ainsi, deux secondes d'un électrogramme mémorisé sous forme comprimée, donnant une vue détaillée "au téléobjectif' d'une arythmie, occupent une page mémoire de 256 mots de huit bits. La technique précitée des chaînes de marqueurs offrent une vue "normale", intermédiaire, une chaîne de marqueurs couvrant approximativement une minute occupant une page de mémoire.

Cette limitation devient particulièrement contraignante dans le cas des appareils de traitement des troubles tachycardiques (cardioverteurs et défibrillateurs) dans la mesure où, dans ces appareils, il est important de pouvoir déterminer rétrospectivement les circonstances du déclenchement d'une thérapie et, ensuite, d'analyser les résultats de cette thérapie, ce qui implique de disposer d'un enregistrement précis des instants précédant et suivant le déclenchement de la thérapie (typiquement, les deux secondes précédant et les deux secondes suivant le choc de cardioversion ou de défibrillation). Mais, pour déterminer l'efficacité sur le long terme des thérapies appliquées par l'appareil implanté, il serait souhaitable de disposer d'informations sur le rythme cardiaque entre deux séances de consultations par le patient, avec des données plus analytiques que celles obtenues par la lecture des histogrammes mémorisés dans l'appareil implanté.

Pour cette raison, l'un des buts de la présente invention est de proposer une fonction Holter perfectionnée pour un appareil, notamment implantable, de surveillance et/ou de thérapie des arythmies, dans lequel le perfectionnement consiste à mémoriser à plusieurs niveaux de détail des informations relatives à un épisode cardiaque, un niveau croissant de détail correspondant à une période d'enregistrement plus courte. En reprenant l'analogie photographique, on peut considérer que le perfectionnement de l'invention consiste à offrir, grâce à un seul et même objectif "zoom", une possibilité de focales multiples.

Un autre but de la présente invention est de proposer une nouvelle fonction Holter implantable, que l'on appellera "compte-rendu d'événement", qui enregistre des événements datés mais survenant moins souvent que les événements des chaînes de marqueurs, permettant ainsi, pour un même espace mémoire, de couvrir une durée plus longue qu'avec une chaîne de marqueurs, les compte-rendus d'événement donnant une vision "au grand angle" de l'activité cardiaque.

Un autre but encore de la présente invention est de permettre un affichage de la relation temporelle entre enregistrements effectués à différents niveaux de détail.

Un autre but encore de la présente invention est de proposer une logique permettant d'arbitrer, entre plusieurs épisodes d'arythmie, celui que l'appareil conservera en mémoire.

Un autre but encore de la présente invention est de permettre à un appareil externe de programmation de réserver sélectivement des sections de mémoire pour d'autres utilisations que des fonctions Holter, par exemple pour mémoriser des programmes.

Pour atteindre ces buts, l'invention propose un appareil du type précité comportant des fonctions d'enregistrement Holter, comme enseigné par le US-A-4 295 474 précité comprenant en outre les moyens énoncés dans la partie caractérisante de la revendication 1.

Les sous-revendications énoncent un certain nombre de caractéristiques subsidiaires avantageuses.

D'autres caractéristiques et avantages de l'invention apparaîtront à la lecture de la description détaillée ci-dessous, faite en référence aux dessins annexés.

La figure 1 est un schéma général montrant les principaux organes de l'appareil de l'invention et les flux de données entre ces éléments.

La figure 2 illustre un exemple d'affichage d'écran du programmateur pour le choix des enregistrements Holter implantables à afficher.

La figure 3 donne un exemple d'un affichage de compte-rendu d'événement.

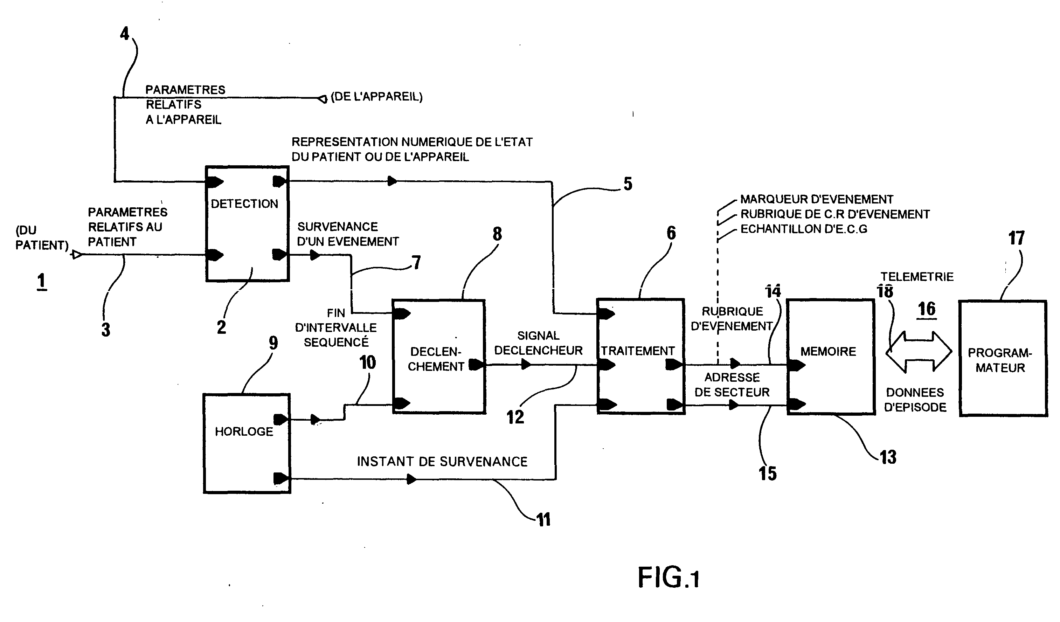

La figure 1 montre les éléments d'un appareil médical conçu pour être implanté dans le corps 1 d'un patient, ainsi que son programmateur externe associé.

L'appareil comporte des moyens de détection 2, pour l'acquisition d'informations 3 concernant le patient 1 et pour l'acquisition d'informations 4 concernant le fonctionnement de l'appareil lui-même. Les moyens de détection 2 convertissent les informations 3 et 4 en une représentation numérique 5 pour traitement par des moyens processeurs 6. Les moyens de détection 2 produisent également des informations 7 permettant à des moyens déclencheurs 8 de déterminer la survenance d'événements prédéterminés concernant l'état du patient ou de l'appareil et discriminer ces événements selon leur nature.

L'appareil comporte également des moyens 9 formant horloge, que l'on appellera "horloge Holter", pour mesurer les intervalles entre événements et pour le séquencement d'intervalles prédéterminés. L'horloge 9 possède une sortie 10 pour signaler aux moyens déclencheurs 8 le moment où survient la fin d'un intervalle séquencé. L'horloge 9 possède une seconde sortie 11 qui permet aux moyens processeurs 6 de connaître la date et l'heure courantes (fonction d'horloge temps réel).

Les moyens déclencheurs 8 délivrent aux moyens processeurs 6 un signal déclencheur 12 lorsque les moyens détecteurs 2 signalent la survenance d'un événement prédéterminé par le signal 7, ou lorsque l'horloge 9 signale la fin d'un intervalle séquencé par le signal 10. Le signal déclencheur 12 contient suffisamment d'informations pour identifier le type d'événement prédéterminé.

L'appareil comporte également une mémoire 13, que l'on appellera "mémoire Holter", partitionnée de la manière qui sera décrite plus bas. Cette mémoire reçoit des données d'événement 14 en provenance des moyens processeurs 6, qui indiquent également à la mémoire, par une adresse 15, l'emplacement où ces données d'événement doivent être mémorisées.

Il est également prévu une liaison de télémétrie 16 permettant à un programmateur externe 17 d'interroger (flèche 18), pour analyse et affichage, les données conservées dans la mémoire.

Les moyens processeurs 6 combinent l'information numérique 5 concernant l'état du patient ou de l'appareil (en provenance des moyens détecteurs 2) avec l'identification du type d'événement donnée par le signal déclencheur 12 et avec l'information 11 de date et heure courantes (en provenance de l'horloge 9) afin de composer une rubrique d'événement 14 à enregistrer dans la mémoire 13. Ils déterminent également l'adresse mémoire 15 où cette rubrique devra être mémorisée.

On peut si nécessaire prévoir des moyens déclencheurs multiples (non illustrés sur la figure) pour permettre un déclenchement sur différents événements prédéterminés, et prévoit également des moyens processeurs multiples (qui ne sont pas, non plus, représentés) pour produire différents types de rubriques d'événement en réponse à un ou plusieurs signaux déclencheurs. Le principe de fonctionnement reste cependant le même que celui exposé précédemment.

On va maintenant considérer trois exemples de types de rubriques d'événement 14:
― Échantillon d'électrogramme : cette rubrique consiste en une représentation numérique du signal de tension instantanée mesurée par les électrodes placés directement sur le coeur. Le nombre d'échantillons par seconde peut être réduit par divers algorithmes de compression déjà décrits dans la littérature. L'appareil peut mémoriser ces échantillons à intervalles fixes ou à intervalles variables.
― Marqueur d'événement : cette rubrique consiste en un code numérique indiquant le type d'événement marqué, accompagné d'un code numérique indiquant l'instant de survenance de l'événement. L'appareil mémorise des marqueurs d'événement pour des événements de stimulation et des événements de détection.
― Compte-rendu d'événement : cette rubrique consiste en un code numérique indiquant le type d'événement survenu, un code numérique indiquant au moins un autre paramètre concernant l'état du patient ou l'état de l'appareil, et un code indiquant l'instant de survenance de l'événement. L'appareil mémorise de tels comptes-rendus pour des événements qui surviennent moins fréquemment que les événements de stimulation ou de détection, par exemple l'application d'une thérapie antiarythmique.

Pour permettre l'affichage simultané d'informations concernant des événements à différents niveaux de détail et l'indication de la relation temporelle entre les représentations à différents niveaux de détail, l'appareil doit "dater" les rubriques d'événement, c'est-à-dire qu'il doit mémoriser suffisamment d'informations sur les rubriques d'événement pour qu'un programmateur externe puisse reconstruire un chronogramme. Pour cela, le programmateur externe doit connaître la durée séparant deux rubriques d'événement quelconques.Plusieurs techniques de "datation" sont possibles :
― Lorsque l'appareil enregistre des rubriques à intervalle fixe, par exemple des échantillons d'électrogramme obtenus à périodicité constante ou des rubriques de compte-rendu d'événement enregistrées une fois par minute, il peut alors dater simplement l'un des événements. Le programmateur utilisera alors le fait que l'intervalle temporel entre deux échantillons consécutifs quelconques est fixe.
― Lorsque l'appareil enregistre des rubriques à intervalle variable, il doit dater chaque rubrique ; il peut inscrire la durée écoulée depuis la rubrique précédente, ou inscrire l'heure donnée par l'horloge au moment de la mémorisation de la rubrique.
― Si l'intervalle entre événements ne peut excéder une certaine valeur prédéterminée, l'appareil n'a besoin que de mémoriser certains chiffres de l'heure donnée par l'horloge, à savoir les chiffres significatifs représentatifs d'un temps plus réduit que cette valeur prédéterminée. Par exemple, si les rubriques ne sont pas séparées de plus d'une minute, l'appareil a seulement besoin de mémoriser la seconde à laquelle l'événement est survenu. Le programmeur peut calculer la durée entre événements en connaissant la seconde à laquelle chaque événement est survenu, sachant qu'il s'est écoulé moins d'une minute entre les événements.

On va maintenant donner un exemple d'application du dispositif illustré figure 1 au cas particulier d'un défibrillateur implantable (à cet égard, on utilisera le terme "défibrillateur" pour désigner un quelconque appareil conçu pour terminer une tachyarythmie par une énergie électrique dépassant notablement l'énergie fournie par des stimulateurs cardiaques implantables, cette définition incluant les défibrillateurs/cardioverteurs/stimulateurs implantables et les défibrillateurs/stimulateurs implantables).

Cette application, si elle utilise de façon particulièrement avantageuse les fonctions enseignées par l'invention, n'est cependant pas limitative et, comme on l'a indiqué plus haut, les enseignements de l'invention peuvent être appliqués à des fonctions Holter incorporées à des stimulateurs simples, ou même à des enregistreurs Holter externes.

Dans cet exemple, les moyens détecteurs 2 de la figure 1 comportent des circuits et un logiciel de détection des arythmies, en eux-mêmes connus, qui détectent et rendent compte du rythme cardiaque. L'horloge 9 est une horloge temps réel qui possède une résolution de 15,6 ms sur cinq octets chacun. L'horloge Holter présente ainsi les caractéristiques suivantes :

| octet | résolution | couverture |
|---|---|---|
| 4 | 2,13 années | 545 années |
| 3 | 3,03 jours | 2,13 années |
| 2 | 17,1 minutes | 3,03 jours |
| 1 | 4,00 secondes | 17,1 minutes |
| 0 | 15,6 millisecondes | 4,00 secondes |

L'horloge 9 comporte également deux séquenceurs (en eux-mêmes connus) délivrant régulièrement des signaux 10 indiquant la fin d'un intervalle séquencé. L'un des séquenceurs délivre un signal toutes les 7,81 ms pour indiquer le moment où il y a lieu de mémoriser un échantillon d'électrogramme ; l'autre séquenceur délivre un signal chaque minute pour indiquer le moment où l'on doit mémoriser certaines rubriques de compte-rendu d'événement (que l'on appellera "compte-rendu minute").

Il est prévu une partition de la mémoire 13 afin de permettre la mémorisation des rubriques d'événement par groupes.

La mémoire est segmentée en un certain nombre d'enregistrements, chaque enregistrement correspondant à un épisode. L'épisode, qui ne couvre qu'une seule arythmie cardiaque, comprend la détection de l'arythmie, l'application d'une thérapie (éventuellement répétée) et la confirmation de la "conversion" réussie de l'arythmie. On appellera "enregistrement courant" l'enregistrement que l'appareil inscrit à un instant donné.

Chaque enregistrement est en outre segmenté en pages, chaque page contenant un seul type de rubrique d'événement. Chaque enregistrement comporte cinq pages de 256 octets, les cinq pages contenant:
― un compte-rendu d'événement pour la totalité de l'arythmie, consistant en une séquence de rubriques de compte-rendu d'événement ;
― un premier électrogramme ("électrogramme d'initiation"), pris sur une durée allant jusqu'à deux secondes avant que l'appareil ne recoure à une thérapie, consistant en une séquence d'échantillons d'électrogramme ;
― une première chaîne de marqueurs mémorisés à la fin de l'électrogramme d'initiation, consistant en une séquence de marqueurs d'événement ;
― un second électrogramme ("électrogramme de conversion"), pris sur une durée allant jusqu'à deux secondes après que la conversion ait été considérée comme réussie ;
― une seconde chaîne de marqueurs mémorisés à la fin de l'électrogramme de conversion.

Pour la gestion de la mémoire Holter, le logiciel des moyens de traitement 6 mémorise dans la mémoire 13, en un emplacement distinct des enregistrements :
― un index pointant sur l'enregistrement courant, et
― un drapeau, contrôlé par le programmateur, permettant d'activer ou désactiver la sélection par l'appareil d'un nouvel enregistrement en tant qu'enregistrement courant ; de cette manière, le programmateur peut s'assurer que l'appareil n'est pas en train d'inscrire un enregistrement pendant une tentative de lecture par le programmateur.

On mémorise également, pour chaque enregistrement, les informations suivantes :
― "Datation" : valeur de l'horloge Holter au moment où l'appareil sauvegarde l'enregistrement, avec une précision de 4 s ;
― "Réservé" : indication de ce que le programmeur a réservé l'enregistrement pour y mémoriser des instructions de programme et non des données Holter; et
― "Événement" : pour indiquer si l'enregistrement :
   · est effacé (code éventuellement positionné par le programmateur après lecture de l'enregistrement),
   · concerne une tachycardie ventriculaire (TV), l'un des types d'arythmie identifiés par les moyens détecteurs 2,
   · concerne une tachycardie sinusale persistante ou une tachycardie supraventriculaire (TSV), qui sont d'autres types d'arythmie identifiés par les moyens détecteurs 2, ou
   · concerne une fibrillation ventriculaire (FV), qui est encore un autre type d'arythmie.

Maintenant que l'on a exposé la partition en pages de la mémoire Holter 15, on va donner un exemple de la structure interne de chaque page.

Chaque page contient une chaîne de rubriques d'événement. Dans le présent exemple, les compte-rendus d'événement comportent huit octets par rubrique, les électrogrammes en comportent un et les marqueurs, deux ; le premier octet de chaque événement ne peut contenir les codes FF_{H} ou FE_{H}, qui sont utilisés par l'appareil pour marquer le début et la fin d'une chaîne :
― Compte-rendu d'événement : l'appareil sauvegarde huit octets pour chaque événement : le premier octet code le type d'événement, le deuxième mémorise un octet de l'horloge Holter avec une résolution de 4 s (pour déterminer la durée séparant les événements) et les six autres dépendent du type d'événement.
― Marqueurs : l'appareil sauvegarde deux octets pour chaque événement : le code de marqueur d'événement (qui n'est jamais FF_{H} ni FE_{H}) et la durée écoulée depuis le début du cycle de stimulation ventriculaire, par unité de 15,625 ms.
― Électrogramme : l'appareil sauvegarde toutes les 7,81 ms un octet représentant l'amplitude de l'électrogramme, avec une étendue de -32 à +31 unités, chaque unité correspondant à un pas d'environ 0,30 mV.

Au début d'une chaîne, l'appareil inscrit FF_{H} sur le premier octet du premier événement et positionne le pointeur sur la deuxième rubrique de la page, pour indiquer l'emplacement où le prochain événement sera inscrit. En fin de chaîne, l'appareil inscrit FE_{H} sur le premier octet de l'événement suivant.

Lors de la lecture de la page, si le premier octet contient FF_{H} et si le premier octet de la deuxième rubrique contient FE_{H} la chaîne est vide ; sinon, la chaîne commence à la deuxième rubrique et se termine à la rubrique précédant celle marquée FE_{H}. Si le premier octet ne contient pas FF_{H}, la chaîne commence à la rubrique suivant celle marquée FE_{H} et se termine à la rubrique précédente.

On a ainsi décrit la structure de l'horloge 9 et de la mémoire 13. On va maintenant décrire un exemple du fonctionnement des moyens détecteurs 2, des moyens déclencheurs 8 et des moyens processeurs 6 lors de la mémorisation d'un épisode en mémoire. Ces moyens sont mis en oeuvre dans les appareils implantables par des techniques en elles-mêmes connues, notamment par logiciel et microprocesseur, ou par des circuits VLSI avec des machines à états spécifiques.
Étape 1 : Lorsque les moyens détecteurs 2 signalent aux moyens déclencheurs 8, via le signal 7, que le rythme cardiaque s'est modifié en une tachyarythmie, les moyens déclencheurs 8 envoient aux moyens processeurs 6 un signal déclencheur 12 indiquant le type d'arythmie. Tout d'abord, les moyens processeurs 6 mémorisent un compte-rendu d'événement et des marqueurs d'initiation pour cette nouvelle arythmie. Ils reçoivent des données 5 d'événement (par exemple l'initiation d'une thérapie antiarythmique) et de marqueur en provenance des moyens détecteurs 2, qui ont reçu du patient certaines données 3 et de l'appareil lui-même d'autres données 4.
Étape 2 : Lorsque les moyens détecteurs ont déterminé qu'il pourrait rester moins de deux secondes avant de recourir à une thérapie (début de stimulation antitachycardique ou début de charge des condensateurs de choc pour application d'une thérapie de cardioversion ou de défibrillation), les moyens déclencheurs 8 indiquent aux moyens processeurs 6, via un nouveau signal déclencheur 12, qu'il y a lieu de commencer l'enregistrement de l'électrogramme d'initiation ; les moyens processeurs 6 reçoivent des moyens détecteurs 2 les données d'électrogramme 5.
Étape 3 : Si, ensuite, les moyens détecteurs déterminent qu'il reste plus de deux secondes avant le recours à une thérapie, ils en informent les moyens déclencheurs, qui à leur tour informent les moyens processeurs de revenir à la détection, de ne pas sauvegarder l'enregistrement en cours mais, au contraire, de se préparer à sauvegarder la présente arythmie ou la suivante dans le même enregistrement. Sinon, si les moyens détecteurs engagent le recours à la thérapie, on continue alors de la manière décrite ci-dessous (dans la description ci-dessous, les moyens détecteurs continuent à délivrer des signaux de commande et de données aux moyens déclencheurs et processeurs de la manière exposée plus haut).
Étape 4 : On mémorise temporairement l'événement qui a déclenché l'arythmie : FV, rythme rapide soutenu ou TV. L'appareil mémorise cet événement en tant que type d'événement après conversion réussie de l'arythmie. On cesse d'enregistrer l'électrogramme d'initiation et on marque sa fin. On marque la fin des marqueurs d'initiation et on commence à mémoriser les marqueurs de conversion. Comme l'appareil arrête l'électrogramme et les marqueurs au même moment, le programmateur saura les aligner pour les afficher concurremment.
Étape 5 : Après détection d'une conversion réussie, on commence à compter quatre cycles d'électrogramme de conversion. Si, au cours de ces quatre cycles, on détecte une nouvelle arythmie et il reste moins de deux secondes avant de recourir à une thérapie pour cette nouvelle arythmie, on passe directement à l'étape suivante. Dans le cas contraire, on passe à l'étape suivante après avoir enregistré au moins quatre cycles.
Étape 6 : Après avoir enregistré l'électrogramme de conversion, on marque la fin de l'électrogramme de conversion, des marqueurs de conversion et du compte-rendu d'événement. On enregistre la date et le type d'événement.

On sélectionne la rubrique suivante. Si au moins trois rubriques sont_disponibles pour enregistrement Holter, l'appareil mémorisera la dernière FV et le dernier rythme rapide soutenu. Pour sélectionner l'enregistrement suivant, si le programmateur a activé la sélection de l'enregistrement suivant, l'appareil commence par rechercher un enregistrement effacé non réservé ; s'il n'en trouve aucun, il recherche le plus ancien événement non réservé qui ne soit pas une FV isolée ou un rythme rapide soutenu isolé et, s'il n'en trouve toujours pas, il continue en réécrivant sur le dernier enregistrement.

Si le détecteur indique qu'il pourrait recourir à la thérapie d'une nouvelle arythmie dans moins de deux secondes, on passe immédiatement à l'enregistrement du nouvel électrogramme d'initiation ; sinon, on se met en détection de tachyarythmie, en attente de l'arythmie suivante.

On a ainsi décrit la séquence de fonctionnement du système de l'invention.

On va donner ci-dessous des exemples des types d'événements que l'appareil peut mémoriser comme marqueur d'événement et compte-rendu d'événement :

Marqueurs de stimulation : l'appareil marque les événements suivants dans les chaînes de marqueurs : P en période réfractaire, détection P, stimulation A, R en période réfractaire, détection R, stimulation V.

Compte-rendu minute : chaque minute l'appareil mémorise les informations suivantes :
― code pour indiquer que le compte-rendu est un compte-rendu minute (80_{H}),
― nombre de détections ventriculaires (y compris les extrasystoles ventriculaires dissociées),
― nombre de détections auriculaires ou de détections auriculaires en période réfractaire,
― nombre d'extrasystoles ventriculaires,
― cycle ventriculaire le plus court,
― moyenne des quatre derniers cycles ventriculaires,
― (zéro).

Compte-rendu de détection des arythmies : chaque fois que le rythme détecté change, l'appareil mémorise une rubrique de compte-rendu d'événement avec les informations suivantes :
― code pour indiquer que le compte-rendu concerne une détection (40_{H}),
― type de rythme,
― corrélation PR (qui est une caractéristique de la détection),
― corrélation RR (qui est une autre caractéristique de la détection),
― type d'accélération cardiaque,
― classification du dernier cycle,
― moyenne des quatre derniers intervalles RR.

Compte-rendu de stimulation antitachycardique : après avoir délivré une séquence de stimulation antitachycardique, juste avant de passer à la redétection, on mémorise les informations suivantes :
― code pour indiquer que le compte-rendu concerne une stimulation antitachycardique (20_{H}),
― numéro de programme de stimulation,
― nombre de séquences de stimulation délivrées,
― dernier intervalle d'échappement,
― nombre de cycles dans la dernière séquence délivrée,
― nombre de cycles dans lesquels il y a eu détection ventriculaire,
― (zéro).

Compte-rendu de délivrance de choc : après délivrance de chaque choc, on mémorise les informations suivantes :
― numéro du programme,
― nombre de chocs délivrés par ce programme,
― (tension en fin de choc -14,2 V) / 25 V,
― (tension stockée après charge ― 14,2 V) / 25 V,
― somme des durées des phases de choc / 0,24 ms,
― '1' si la forme de l'onde de choc était à "tilt" constant, sinon '0',
― durée de charge / 0,25 s.

Le système de l'invention comporte également une liaison de télémétrie 18 vers un programmateur externe référencé 17.

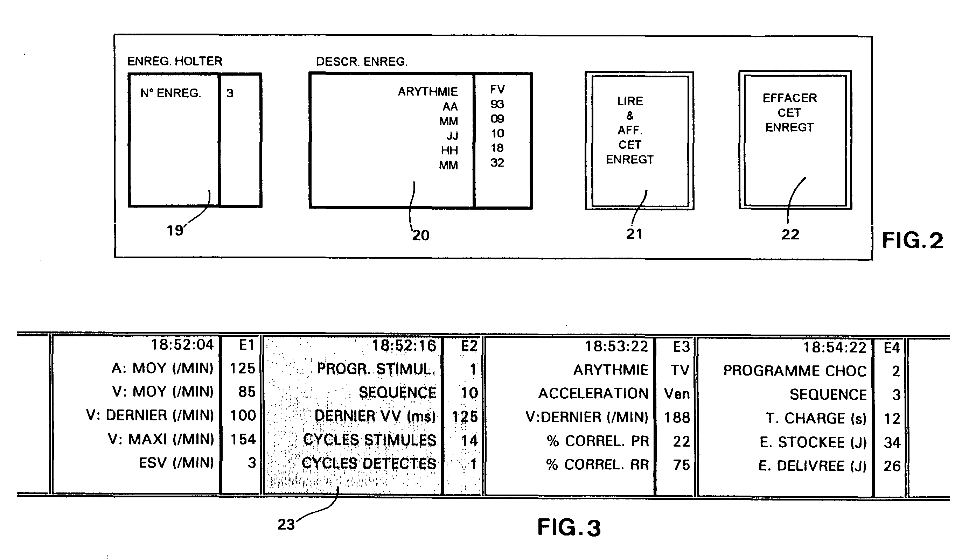

La figure 2 illustre un exemple d'un écran de programmateur permettant de choisir l'affichage d'un enregistrement Holter.

L'écran affiche en 19 le numéro d'enregistrement sélectionné en cours et en 20 une brève description de l'enregistrement. Il affiche aussi en 21 et 22 des "boutons" permettant à l'utilisateur de lire et d'afficher l'enregistrement choisi, ou de l"'effacer" (c'est-à-dire de libérer l'enregistrement pour permettre à l'appareil implanté de réécrire dessus, comme expliqué *supra*).

L'écran d'affichage Holter correspondant à un enregistrement nécessite la présentation de trois types d'informations : les compte-rendus d'événement, les chaînes de marqueurs correspondant au début et à la fin de l'arythmie et les images des électrogrammes correspondant au début et à la fin de l'arythmie.

L'en-tête de l'écran peut par exemple donner le numéro d'enregistrement, le type d'arythmie, la date et l'heure, par exemple : Enr. Holter n°3, FV, 10.09.93,11:24

Le programmateur peut afficher les rubriques successives de chaque compte-rendu d'événement sous la forme d'un ruban en haut de l'écran.

La première ligne de chaque rubrique affichée sur ce ruban 23 (figure 3) donne l'instant de survenance de l'événement (avec une résolution de 4 s) et le numéro d'événement (E1 étant le premier événement mémorisé dans le compte-rendu, etc.), les chaînes de marqueurs utilisant ce numéro d'événement pour indiquer dans quel cycle l'événement est survenu.

Sur le même écran, on peut afficher concurremment, dans un ruban au centre de l'éc:ran, les chaînes de marqueurs (à 25 mm/s) lorsqu'elles sont disponibles et, dans un ruban au bas de l'écran, l'électrogramme (également à 25 mm/s) lorsqu'il est disponible. Il est possible d'afficher une échelle temporelle entre les marqueurs et l'électrogramme. Si l'on doit afficher l'électrogramme ou les marqueurs, une échelle temporelle horizontale fixe est nécessaire et il faut donc prévoir un espacement horizontal entre les rubriques de compte-rendu d'événement.

Comme il y a un marqueur de la chaîne pour chaque compte-rendu d'événement, l'affichage peut indiquer sur la chaîne de marqueurs à quel cycle exactement chaque compte-rendu d'événement est survenu. Une manière commode de le faire pourrait consister à afficher au-dessus de la chaîne de marqueurs une ligne avec le numéro d'événement du cycle correspondant.

L'utilisateur peut faire avancer et reculer dans le temps l'affichage avec les touches de clavier "flèche à droite " et "flèche à gauche". Lorsqu'il n'y a pas de d'électrogramme ou de marqueur affiché, ces touches provoquent un déplacement à la rubrique suivante du compte-rendu d'événement. Lorsqu'un électrogramme ou un marqueur sont présents, les touches provoquent un déplacement d'une seconde vers la droite ou vers la gauche.

## Revendications

1. Un appareil médical à fonctions d'enregistrement Holter, comprenant :
― des moyens détecteurs (2), pour recueillir des informations (3, 4) sur l'état du patient et/ou de l'appareil et pour convertir ces informations en des données numériques représentatives (5) ;
― des moyens formant horloge (9), produisant au moins un signal d'horloge (10, 11) définissant une donnée temporelle ;
― des moyens formant mémoire adressable (13), pour la mémorisation durable d'informations, cette mémoire étant partitionnée en secteurs pour la mémorisation de rubriques distinctes ;
― des moyens déclencheurs (8), fonctionnant en réponse aux moyens détecteurs et/ou aux moyens formant horloge, pour reconnaître la survenance d'au moins un type prédéterminé d'événement et pour produire dans ce cas un signal déclencheur (12) représentatif du type prédéterminé d'événements ; et
― des premiers moyens de traitement (6), fonctionnant en réponse à au moins une valeur prédéterminée du signal déclencheur, pour combiner les données provenant des moyens détecteurs, les données provenant des moyens formant horloge et le signal déclencheur afin de constituer un enregistrement d'événement à un premier niveau de détail, couvrant une première période temporelle, et pour mémoriser cet enregistrement d'événement dans un premier secteur de la mémoire,
caractérisé par :
― des seconds moyens de traitement (6), fonctionnant en réponse à au moins une valeur prédéterminée du signal déclencheur, pour combiner les données provenant des moyens détecteurs, les données provenant des moyens formant horloge et le signal déclencheur afin de constituer un enregistrement d'événement à un second niveau de détail, couvrant une seconde période temporelle, et pour mémoriser cet enregistrement d'événement dans un second secteur de la mémoire,
le second niveau de détail comportant moins de détails que le premier niveau et la seconde période temporelle étant plus étendue que la première période temporelle.

2. L'appareil de la revendication 1, dans lequel les seconds moyens de traitement mémorisent un compte-rendu d'événement comprenant une pluralité de rubriques de compte-rendu d'événement pour rendre compte d'événements survenant moins fréquemment que les événements de stimulation et de détection, chaque rubrique de compte-rendu d'événement contenant un code représentatif du type d'événement, au moins un paramètre supplémentaire représentatif de l'état du patient ou de l'appareil, et une information indiquant l'instant de survenance de l'événement.

3. L'appareil de la revendication 2, dans lequel l'un des types prédéterminés d'événement est l'écoulement d'un intervalle de temps prédéterminé mesuré par l'horloge, pour l'enregistrement de rubriques de compte-rendu d'événement à une périodicité constante prédéterminée.

4. L'appareil de la revendication 3, dans lequel le paramètre supplémentaire représentatif de l'état du patient ou de l'appareil comprend la valeur, sur ledit intervalle de temps prédéterminé, d'au moins l'un des paramètres du groupe comprenant : le nombre de battements ventriculaires détectés, le nombre de battements auriculaires détectés, le nombre d'extrasystoles ventriculaires détectées, la plus courte longueur de cycle ventriculaire et la longueur moyenne de cycle ventriculaire sur le dernier intervalle de temps.

5. L'appareil de la revendication 2, dans lequel, les moyens détecteurs classant le rythme cardiaque en catégories comprenant un rythme sinusal et une ou plusieurs classes de tachyarythmie, l'un des types prédéterminés d'événement comprend un changement de classification du rythme cardiaque détecté par l'appareil et le paramètre supplémentaire représentatif de l'état du patient ou de l'appareil comprend au moins l'un des paramètres du groupe comprenant : la classe du rythme et les critères de classification, ces derniers comprenant au moins l'un des paramètres du groupe comprenant : la fréquence, la fréquence moyenne, l'accélération et la stabilité du rythme, ainsi que l'association auriculo-ventriculaire.

6. L'appareil de la revendication 2, dans lequel, l'appareil étant susceptible d'appliquer une thérapie de stimulation antitachycardique, l'un des types prédéterminés d'événement est l'application d'une séquence de stimulation antitachycardique au patient.

7. L'appareil de la revendication 6, dans lequel le paramètre additionnel relatif à l'état du patient ou de l'appareil comprend au moins l'un des paramètres du groupe comprenant : l'identification du programme et de la séquence de stimulation antitachycardique, le nombre de séquences délivrées, le dernier intervalle d'échappement, le nombre de cycles dans la dernière séquence délivrée et le nombre de cycles au cours desquels est survenu une détection ventriculaire.

8. L'appareil de la revendication 2, dans lequel, l'appareil comportant des moyens défibrillateurs, l'un des types prédéterminés d'événement est l'application d'une thérapie de choc au patient et le paramètre additionnel relatif à l'état du patient ou de l'appareil comprend au moins l'un des paramètres du groupe comprenant : le numéro du programme de choc, le nombre de chocs délivré par le programme, la tension en fin de choc, la tension initiale de choc, la durée du choc, la forme d'onde du choc et le temps de charge.

9. L'appareil de la revendication 8, dans lequel les types prédéterminés d'événements comprennent au moins un événement du groupe comprenant : la survenance d'une fibrillation ou d'une tachycardie nécessitant une thérapie de cardioversion, la survenance d'une tachycardie nécessitant une thérapie par stimulation antitachycardique, l'application d'une thérapie de cardioversion et l'application d'une thérapie de stimulation antitachycardique.

10. L'appareil de la revendication 1, dans lequel l'un des moyens de traitement mémorise une chaîne de marqueurs d'événement pour le marquage d'événements de stimulation et de détection, chaque marqueur d'événement comprenant un code correspondant au type d'événement et un code temporel tel que des moyens programmateurs externes puissent déterminer l'instant de survenance de chaque événement marqué.

11. L'appareil de la revendication 1, dans lequel les premiers moyens de traitement mémorisent un électrogramme et les seconds moyens de traitement mémorisent une chaîne de marqueurs.

12. L'appareil de la revendication 1, dans lequel les premiers moyens de traitement mémorisent une chaîne de marqueurs et les seconds moyens de traitement mémorisent un compte-rendu d'événement.

13. L'appareil de la revendication 1, dans lequel les premiers moyens de traitement mémorisent un électrogramme et les seconds moyens de traitement mémorisent un compte-rendu d'événement.

14. L'appareil de la revendication 1, dans lequel il est en outre prévu des troisièmes moyens de traitement, fonctionnant en réponse à au moins une valeur prédéterminée du signal déclencheur, pour combiner les données provenant des moyens détecteurs, les données provenant des moyens formant horloge et le signal déclencheur afin de constituer un enregistrement d'événement à un troisième niveau de détail, couvrant une troisième période temporelle, et pour mémoriser cet enregistrement d'événement dans un troisième secteur de la mémoire,
le troisième niveau de détail comportant moins de détails que le second niveau et la troisième période temporelle étant plus étendue que la seconde période temporelle,
et dans lequel les premiers moyens de traitement mémorisent un électrogramme, les seconds moyens de traitement mémorisent une une chaîne de marqueurs, et les troisièmes moyens de traitement mémorisent un compte-rendu d'événement.

15. L'appareil de la revendication 1, dans lequel l'appareil est un appareil implantable et il est prévu en outre :
― des moyens de télémétrie (16), pour permettre la lecture et l'affichage des informations conservées dans la mémoire adressable (13) par un programmateur externe (17) et pour permettre la programmation de l'appareil au moyen de ce programmateur, et
― des moyens sélecteurs (6), pour sélectionner, après inscription dans l'un des secteurs de la mémoire des informations relatives à un événement, le secteur de la mémoire où seront inscrites les informations relatives à l'épisode suivant, ces moyens sélecteurs comprenant :
· des moyens de verrouillage d'adresse, pour empêcher, sur ordre du programmateur, de modifier le contenu d'un secteur adressé courant afin que le programmateur puisse lire de tels secteurs sans risque que l'appareil ne vienne y écrire pendant la durée de la lecture par le programmateur,
· des moyens pour définir un secteur comme "réservé", pour empêcher sa sélection en vue de la mémorisation d'informations relatives à un événement,
· des moyens pour définir un secteur comme "non effacé", pour empêcher sa sélection lorsqu'ils contient un événement mémorisé non encore lu par le programmateur,
· des moyens pour définir un secteur comme "prioritaire", pour empêcher sa sélection lorsqu'il contient un événement d'un type particulier considéré comme devant être sauvegardé, et · des moyens pour sélectionner un secteur en fonction de l'ancienneté de l'événement qui y est mémorisé, de manière à pouvoir remplacer le plus ancien des événements par ledit événement suivant.

16. L'appareil de la revendication 15, dans lequel ledit événement est la survenance d'une fibrillation ou d'une tachycardie et l'application d'une thérapie de cardioversion, ou bien la survenance d'une tachycardie et l'application d'une thérapie de stimulation antitachycardique.

17. L'appareil de la revendication 15, dans lequel les moyens sélecteurs, lorsqu'ils sont activés par le programmateur externe, exécutent les étapes consistant à :
a) chercher à sélectionner un secteur qui ne soit pas défini comme "non effacé" ou "réservé",
b) dans la négative, chercher à sélectionner le secteur contenant le plus ancien des événements et qui ne soit pas défini comme "prioritaire", et
c) dans la négative, sélectionner le secteur contenant le dernier événement inscrit.

## Claims

1. A medical apparatus having Holter recording functions, the apparatus comprising:
- detector means (2) for picking up information (3, 4) concerning the state of the patient and/or of the apparatus and for converting said information into representative digital data (5);
- clock-forming means (9) producing at least one clock signal (10, 11) defining time data;
- addressable memory-forming means (13) for durable storage of information, said memory being partitioned into sectors for storing distinct entries;
- trigger means (8) responsive to the detector means and/or to the clock-forming means to recognize the occurrence of at least one predetermined type of event and to respond thereto by producing a trigger signal (12) representative of the predetermined type of event; and
- first processor means (6) operating in response to at least one predetermined value of the trigger signal to combine the data coming from the detector means, the data corning from the clock forming means, and the trigger signal so as to make up an event record at a first level of detail covering a first period of time, and so as to store said event record in a first sector of the memory;
the apparatus being characterized by:
- second processor means (6) responsive to at least one predetermined value of the trigger signal to combine the data coming from the detector means, the data coming from the clock-forming means, and the trigger signal so as constitute an event record at a second level of detail, covering a second time period, and so as to store said event record in a second memory sector,
the second level of detail including less detail than the first level and the second time period being longer than the first time period.

2. The apparatus of claim 1, in which the second processor means store an event log comprising a plurality of event log entries to log events that occur less frequently than stimulation and detection events, each event log entry containing a code representative of the type of event, at least one additional parameter representative of the state of the patient or of the apparatus, and information indicating the instant at which the event occurred.

3. The apparatus of claim 2, in which one of the predetermined types of event is a predetermined time interval as measured by the clock elapsing so as to record event log entries at a predetermined constant frequency.

4. The apparatus of claim 3, in which the additional parameter representative of the state of the patient or of the apparatus comprises the value, over said predetermined time interval, of at least one of the parameters in the group comprising: the number of ventricular beats detected; the number of auricular beats detected; the number of ventricular extrasystoles detected; the shortest length of the ventricular cycle; and the mean length of the ventricular cycle over the latest time interval.

5. The apparatus of claim 2, in which the detector means classify the heart rhythm into categories comprising a sinus rhythm and one or more classes of tachyarrhythmia, one of the predetermined types of event comprising a change in the classification of the heart rhythm detected by the apparatus and the additional parameter representative of the state of the patient or of the apparatus comprises at least one of the parameters in the group comprising: the class of the rhythm and the classification criteria; said criteria comprising at least one of the parameters in the group comprising: frequency, mean frequency, acceleration, and stability of the rhythm, and also auriculo-ventricular association.

6. The apparatus of claim 2, in which, for apparatus capable of applying anti-tachycardial stimulation therapy, one of the predetermined types of event is the application of an anti-tachycardial stimulation sequence to the patient.

7. The apparatus of claim 6, in which the additional parameter relating to the state of the patient or of the apparatus comprises at least one of the parameters in the group comprising: identity of the anti-tachycardial stimulation program and sequence; the number of sequences delivered; the lastest escape interval; the number of cycles in the lastest delivered sequence; and the number of cycles during which a ventricular detection has occurred.

8. The apparatus of claim 2, in which the apparatus includes defribillator means, one of the predetermined types of event is the application of shock therapy to the patient and the additional parameter relating to the state of the patient or of the apparatus comprises at least one of the parameters in the group comprising: the number of the shock program; the number of shocks delivered by the program; the end-of-shock voltage; the initial shock voltage; the duration of the shock; the waveform of the shock; and charging time.

9. The apparatus of claim 8, in which the predetermined event types comprise at least one event in the group comprising: the occurrence of fibrillation or of tachycardia requiring cardioversion therapy; the occurrence of tachycardia requiring therapy by anti-tachycardial stimulation; the application of cardioversion therapy; and the application of anti-tachycardial stimulation therapy.

10. The apparatus of claim 1, in which one of the treatment means stores a chain of event markers for marking stimulation and detection events, each event marker comprising a code corresponding to the type of event and a time code enabling external programmer means to determine the instant at which each marked event occurred.

11. The apparatus of claim 1, in which the first treatment means store an electrogram and the second treatment means store a marker chain.

12. The apparatus of claim 1, in which the first treatment means store a marker chain and the second treatment means store an event log.

13. The apparatus of claim 1, in which the first treatment means store an electrogram and the second treatment means store an event log.

14. The apparatus of claim 1, in which third treatment means are also provided responsive to at least one predetermined value of the trigger signal to combine the data coming from the detector means, the data coming from the clock forming means, and the trigger signal, so as to make up an event record at a third level of detail, covering a third time period, and so as to store said event record in a third sector of the memory,
the third level of detail including less detail than the second level, and the third time period being longer than the second time period,
and in which the first processor means store an electrogram, the second processor means store a marker chain, and the third processor means store an event log.

15. The apparatus of claim 1, in which the apparatus is an implantable apparatus and the following are provided:
- telemetry means (16) enabling information stored in the adjustable memory (13) to be read and displayed by an external programmer (17) and enabling the apparatus to be programmed by means of said programmer; and
- selector means (6) for operating after information relating to an event has been written in one of the sectors of the memory to select the memory sector in which the information relating to the following episode is to be written,
said selector means comprising:
- address-locking means to enable the programmer to prevent the content of a currently addressed sector being modified so as to enable the programmer to read such sectors without any risk of the apparatus writing thereto while the programmer is reading therefrom;
- means for defining a sector as being "reserved" so as to prevent it from being selected to store information relating to an event;
- means for defining a sector as "not erased" so as to prevent it from being selected while it contains an event that has been stored but not yet read by the programmer;
- means for defining a sector as a "priority" sector, to prevent it being selected while it contains an event of a particular type considered as being essential for saving; and
- means for selecting a sector as a function of the age of the event stored therein so as to replace the oldest event by said following event.

16. The apparatus of claim 15, in which said event is the occurrence of fibrillation or of tachycardia, and the application of cardioversion therapy, or the occurrence of tachycardia and the application of anti-tachycardial stimulation therapy.

17. The apparatus of claim 15, in which the selector means, when activated by the external programmer, execute the steps consisting in:
a) attempting to select a sector which is not defined as "not erased" or "reserved";
b) when that is not possible, seeking to select a selector containing the oldest event that is not defined as being a "priority" sector; and
c) when that is not possible, selecting the sector containing the lastest event to be written.

## Patentansprüche

1. Medizinisches Gerät für die Holter-Funktionsaufzeichnung, aufweisend:
- Detektoreinrichtungen (2) zum Empfangen von Informationen (3, 4) über den Zustand des Patienten und/oder des Gerätes, und zum Umwandeln dieser Informationen in repräsentative, numerische Daten (5);
- Einrichtungen, die einen Taktgeber (9) bilden, der mindestens ein einzelnes Taktsignal (10, 11) erzeugt, das eine Zeitangabe definiert;
- Einrichtungen, die einen adressierbaren Speicher (13) zum Speichern dauerhafter Informationen bilden, wobei dieser Speicher für die Speicherung distinkter Rubriken in Sektoren unterteilt ist;
- Auslöseeinrichtungen (8), die als Antwort auf die Detektoreinrichtungen und/oder die den Taktgeber bildenden Einrichtungen wirksam werden, um das Auftreten mindestens eines vorbestimmten Typs von Ereignis zu erkennen, und um in diesem Falle ein Auslösesignal (12) zu erzeugen, das für den vorbestimmten Typ von Ereignissen kennzeichnend ist; und
- erste Behandlungseinrichtungen (6), die als Antwort auf mindestens einen einzelnen vorbestimmten Wert des Auslösesignals wirksam werden, um die Daten, die von den Detektoreinrichtungen kommen, die Daten, die von den den Taktgeber bildenden Einrichtungen kommen, und das Auslösesignal kombinieren, um eine Ereignisaufzeichnung auf einem ersten Detailniveau zu bilden, das eine erste Zeitperiode überdeckt, und um diese Ereignisaufzeichnung in einem ersten Sektor des Speichers zu speichern,
gekennzeichnet durch:
- zweite Behandlungseinrichtungen (6), die als Antwort auf mindestens einen einzelnen vorbestimmten Wert des Auslösesignals wirksam werden, um die von den Detektoreinrichtungen kommenden Daten, die von den den Taktgeber bildenden Einrichtungen kommenden Daten und das Auslösesignal zu kombinieren, um eine Ereignisaufzeichnung auf einem zweiten Detailniveau zu bilden, das eine zweite Zeitperiode abdeckt, und um diese Ereignisaufzeichnung in einem zweiten Sektor des Speichers zu speichern, wobei das zweite Detailniveau weniger Details aufweist als das erste Niveau, und wobei die zweite Zeitperiode länger dauert als die erste Zeitperiode.

2. Gerät nach Anspruch 1, bei dem die zweiten Behandlungseinrichtungen einen Ereignisbericht speichern, der eine Mehrzahl von Ereignisberichtsrubriken umfaßt, um über Ereignisse zu berichten, die weniger häufig als die Ereignisse der Stimulation und der Detektion auftreten, wobei jede Ereignisberichtsrubrik einen Code, der für den Ereignistyp kennzeichnend ist, mindestens einen zusätzlichen Parameter, der für den Zustand des Patienten oder des Gerätes kennzeichnend ist, und eine Information enthält, die für den Zeitpunkt des Auftretens des Ereignisses kennzeichnend ist.

3. Gerät nach Anspruch 2, bei dem einer der vorbestimmten Ereignistypen der Ablauf eines vorbestimmten Zeitintervalls ist, gemessen durch den Taktgeber, zwecks Aufzeichnung der Ereignisberichtsrubriken mit einer vorbestimmten, konstanten Wiederholungsrate.

4. Gerät nach Anspruch 3, bei dem der für den Zustand des Patienten oder des Gerätes kennzeichnende zusätzliche Parameter, über das genannte vorbestimmte Zeitintervall hinweg, mindestens einer der Parameter der Gruppe ist, die aufweist: die Anzahl der detektierten ventrikulären Schläge, die Anzahl der detektierten aurikulären Schläge, die Anzahl der detektierten ventrikulären Extrasystolen, die kürzeste Länge des ventrikulären Zyklus' und die mittlere Länge des ventrikulären Zyklus' während des letzten Zeitintervalls.

5. Gerät nach Anspruch 2, bei dem im Falle, daß die Detektoreinrichtungen den kardialen Rhythmus in Kategorien unterteilen, welche einen sinusalen Rhythmus und eine oder mehrere Tachyarythmieklassen aufweisen, wobei einer der vorbestimmten Ereignistypen eine Änderung der Klassifikation des von dem Gerät detektierten kardialen Rhythmus' umfaßt, und wobei der für den Zustand des Patienten oder des Gerätes kennzeichnende zusätzliche Parameter mindestens einen der Parameter der Gruppe aufweist, die enthält,: die Klasse des Rhythmus' und die Klassifikationskriterien, wobei letztere mindestens einen Parameter der Gruppe aufweist, die enthält: die Frequenz, die mittlere Frequenz, die Beschleunigung und die Stabilität des Rhythmus' sowie den aurikulo-ventrikulären Zusammenhang.

6. Gerät nach Anspruch 2, bei dem im Falle, daß das Gerät in der Lage ist, eine anti-tachykardiale Stimulationstherapie auszuführen, der eine der vorbestimmten Ereignistypen die Anwendung einer anti-tachykardialen Stimulationssequenz beim Patienten ist.

7. Gerät nach Anspruch 6, bei dem der auf den Zustand des Patienten oder des Gerätes bezogene zusätzliche Parameter mindestens einen Parameter derjenigen Gruppe aufweist, die umfaßt: die Identifikation des Programms und der anti-tachykardialen Stimulationssequenz; die Anzahl der verabfolgten Sequenzen; die letzte Intervallhemmung; die Anzahl der Zyklen in der letzten verabfolgten Sequenz und die Anzahl der Zyklen, in deren Verlauf eine ventrikuläre Detektion aufgetreten ist.

8. Gerät nach Anspruch 2, bei dem im Falle, daß das Gerät Defibrilliereinrichtungen umfaßt, einer der vorbestimmten Ereignistypen die Anwendung einer Schocktherapie am Patienten ist und der zusätzliche Parameter in Bezug auf den Zustand des Patienten oder des Gerätes mindestens einen der Parameter derjenigen Gruppe umfaßt, die aufweist: die Nummer des Schockprogramms, die Anzahl der durch das Programm verabfolgten Schocks; die Spannung am Schockende, die Anfangsspannung des Schocks, die Dauer des Schocks, die Wellenform des Schocks und die Entladungsdauer.

9. Gerät nach Anspruch 8, bei dem die vorbestimmten Ereignistypen mindestens ein Ereignis der Gruppe aufweisen, die umfaßt: das Auftreten einer Fibrillation oder einer Tachykardie, die eine Cardioversionstherapie erfordert, das Auftreten einer Tachykardie, die eine Therapie durch anti-tachykardiale Stimulation erfordert, die Anwendung einer Cardioversionstherapie und die Anwendung einer anti-tachykardialen Stimulationstherapie.

10. Gerät nach Anspruch 1, bei dem die eine der Behandlungseinrichtungen eine Kette von Ereignismarkierern zum Markieren von Stimulations- und Detektionsereignissen speichert, wobei jeder Ereignismarker einen Code entsprechend dem Ereignistyp und einen Zeitcode solcher Art umfaßt, daß die externen Programmiereinrichtungen den Zeitpunkt des Auftretens jedes markierten Ereignisses bestimmen können.

11. Gerät nach Anspruch 1, bei dem die ersten Behandlungseinrichtungen ein Elektrogramm speichern, und bei dem die zweiten Behandlungseinrichtungen eine Markerkette speichern.

12. Gerät nach Anspruch 1, bei dem die ersten Behandlungseinrichtungen eine Markiererkette speichern, und bei dem die zweiten Behandlungsmittel ein Berichtsereignis speichern.

13. Gerät nach Anspruch 1, bei dem die ersten Behandlungseinrichtungen ein Elektrogramm speichern, und bei dem die zweiten Behandlungseinrichtungen einen Ereignisbericht speichern.

14. Gerät nach Anspruch 1, bei dem weiter dritte Behandlungseinrichtungen vorgesehen sind, die als Antwort auf mindestens einen einzelnen vorbestimmten Wert des Auslösesignals wirksam werden, um die von den Detektoreinrichtungen kommenden Daten, die von den, den Taktgeber bildenden Einrichtungen kommenden Daten, und das Auslösesignal kombinieren, so daß eine Ereignisaufzeichnung auf einem dritten Detailniveau gebildet wird, das eine dritte Zeitperiode überdeckt, und um diese Ereignisaufzeichnung in einem dritten Sektor des Speichers zu speichern, wobei das dritte Detailniveau weniger Details als das zweite Niveau umfaßt und die dritte Zeitperiode ausgedehnter als die zweite Zeitperiode ist, und wobei die ersten Behandlungseinrichtungen ein Elektrogramm speichern, wobei die zweiten Behandlungseinrichtungen eine Markiererkette speichern, und wobei die dritten Behandlungseinrichtungen einen Ereignisbericht speichern.

15. Gerät nach Anspruch 1, wobei das Gerät ein implantierbares Gerät ist und weiter ausgestattet ist mit:
- Telemetrieeinrichtungen (16), um das Ablesen und die Anzeige von Informationen zu gestatten, welche in dem durch einen externen Programmierer (17) adressierbaren Speicher (13) aufbewahrt werden, und um die Programmierung des Geräts mit Hilfe dieses Programmierers zu ermöglichen, und
- Wähleinrichtungen (6), um nach Einschreiben in einen der Sektoren des Speichers von Informationen in Bezug auf ein Ereignis, den Sektor des Speichers auszuwählen, in welchem die auf eine nachfolgende Episode bezugnehmenden Informationen eingeschrieben sind,
wobei diese Wähleinrichtungen umfassen:
• Einrichtungen zum Sperren einer Adresse, um zu verhindern, auf Befehl des Programmierers den Inhalt eines aktuell adressierten Sektors zu verändern, damit der Programmierer derartige Sektoren lesen kann, ohne Gefahr zu laufen, daß das Gerät während der Dauer der Ablesung durch den Programmierer darin etwas einschreibt,
• Einrichtungen zum Definieren eines Sektors mit "reserviert", um seine Wahl im Hinblick auf die Speicherung von Informationen in Bezug auf ein Ereignis zu verhindern,
• Einrichtungen zum Definieren des Sektors als "nicht gelöscht", um seine Wahl zu verhindern, wenn er ein noch nicht durch den Programmierer gelesenes Ereignis speichert,
• Einrichtungen zum Definieren eines Sektors als "prioritär", um seine Wahl für den Fall zu verhindern, daß er ein Ereignis eines besonderen Typs enthält, das so eingeschätzt wird, daß es aufbewahrt werden muß, und
• Einrichtungen zum Wählen eines Sektors in Abhängigkeit vom Altersrang des Ereignisses, das darin gespeichert ist, derart, daß das ältere der Ereignisse durch das nachfolgende Ereignis ersetzt werden kann.

16. Gerät nach Anspruch 15, bei dem das genannte Ereignis das Auftreten einer Fibrillation oder einer Tachykardie und die Anwendung einer Cardioversionstherapie ist oder aber das Auftreten einer Tachykardie und die Anwendung einer anti-tachykardialen Stimulationstherapie ist.

17. Gerät nach Anspruch 15, bei dem die Wähleinrichtungen, wenn sie durch den externen Programmierer aktiviert werden, Schritte ausführen, die darin bestehen, daß:
a) der Versuch zum Wählen eines Sektors unternommen wird, der nicht als "nicht gelöscht" oder "reserviert" definiert ist,
b) verneinendenfalls versucht wird, denjenigen Sektor zu wählen, der die ältesten Ereignisse enthält und nicht als "prioritär" definiert ist, und
c) verneinendenfalls derjenige Sektor gewählt wird, der das letzte eingeschriebene Ereignis enthält.
